(19)

Europäisches Patentamt
European Patent Office
Office européen des brevets

(11) **EP 1 860 460 A1**

(12) **EUROPEAN PATENT APPLICATION**

(43) Date of publication:
**28.11.2007 Bulletin 2007/48**

(51) Int Cl.:
*G01S 15/89* (2006.01)  *G01S 7/52* (2006.01)
*A61B 8/00* (2006.01)  *G10K 11/34* (2006.01)

(21) Application number: **07010111.8**

(22) Date of filing: **22.05.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **23.05.2006 KR 20060046253**
**25.07.2006 KR 20060069989**

(71) Applicant: **MEDISON CO., LTD.**
**Kangwon-do 250-870 (KR)**

(72) Inventors:
• **Bae, Moo Ho**
**Sincheon-dong, Seoul 138-240 (KR)**

• **Daigle, Ronald E.**
**Washington 98053 (US)**
• **Ahn, Chi Young**
**Gangnam-gu, Seoul 135-280 (KR)**
• **Yoon, Ra Young**
**Gangnam-gu, Seoul 135-280 (KR)**

(74) Representative: **Lorenz, Werner**
**Lorenz & Kollegen**
**Patent- und Rechtsanwaltskanzlei**
**Alte Ulmer Strasse 2**
**89522 Heidenheim (DE)**

(54) **Ultrasound diagnostic system and method for adjusting output of digital signals**

(57)    There is provided an ultrasound system, which includes a probe for providing electrical analog signals in response to ultrasound signals reflected from a target object; an analog-to-digital converter for converting the electrical analog signals to digital signals; and a beam former for extracting a part of digital signals at a rate of n times of a center frequency of the electrical analog signals to form a receive beam, wherein n is a positive integer.

FIG. 8

EP 1 860 460 A1

**Description**

The present application claims priority from Korean Patent Application Nos. 10-2006-0046253 (filed on May 23, 2006) and 10-2006-0069989 (filed on July 25, 2006), the entire subject matters of which are incorporated herein by reference.

**BACKGROUND**

1. **Field**

The present invention generally relates to an ultrasound diagnostic system, and more particularly to an ultrasound diagnostic system and a method for adjusting the amount of digital signals outputted from a beam former.

2. **Background**

An ultrasound diagnostic system transmits ultrasound signals into a target object and receives ultrasound echo signals. The echo ultrasound signals are converted into electrical image signals, thereby providing an internal image of the target object. The ultrasound signals are transmitted and received by a probe. The probe includes transducer elements for reciprocally converting electric signals to ultrasound signals. Since a probe having an array transducer of various types is used, a high-resolution ultrasound image can be acquired.

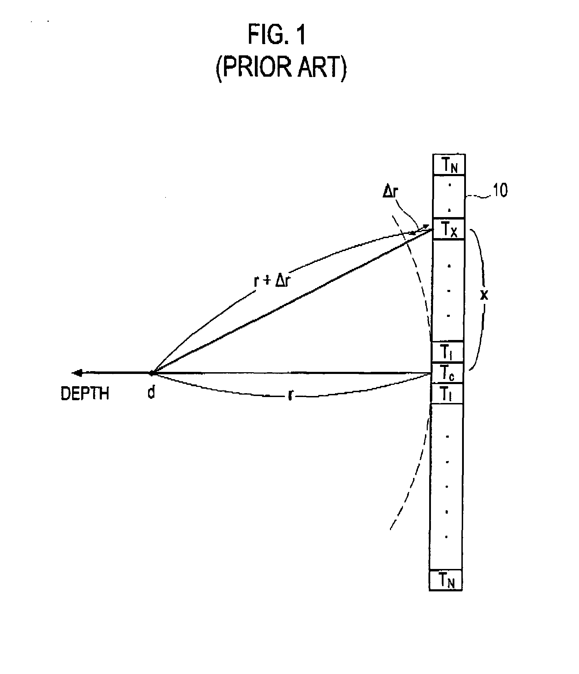

Fig. 1 is a schematic diagram for explaining a transmission/reception focusing method of ultrasound signals in a probe using an array transducer. Referring to Fig. 1, ultrasound signals produced at each transducer element of the array transducer 10 should be focused on a focal point positioned at a depth of d so as to obtain a high-resolution ultrasound image A distance between a center transducer element Tc and the focal point is shortest, whereas a distance between an edge transducer element $T_N$ and the focal point is longest among the distances between each transducer element and the focal point. The ultrasound diagnostic system includes a beam former for forming electrical transmission signals by reflecting distance differences between each transducer clement and the focal point. The beam former forms a plurality of electric transmission signals, i.e., a transmission beam based on a delay profile, which is determined by considering the distance differences between each transducer element and the focal point. The transmission beam is delivered to the array transducer 10 and then converted into ultrasound signals. The ultrasound signals are transmitted to the target object to be focused on the focal point.

Also, the times in which the ultrasound signal reflected from the focal point arrives at each transducer element are different. An ultrasound signal reflected from the focal point travels a distance of "r" to arrive at a center transducer element Tc, while an ultrasound signal reflected from the focal point travels a distance of r+∆r to reach a transducer element Tx. That is, the ultrasound signal received at the transducer element Tx is delayed by ∆r compared to the ultrasound signal received at the transducer element Tc. Therefore, the beam former compensates for the delays of the ultrasound signals received at each transducer element with reference to a position of the center transducer element Tc.

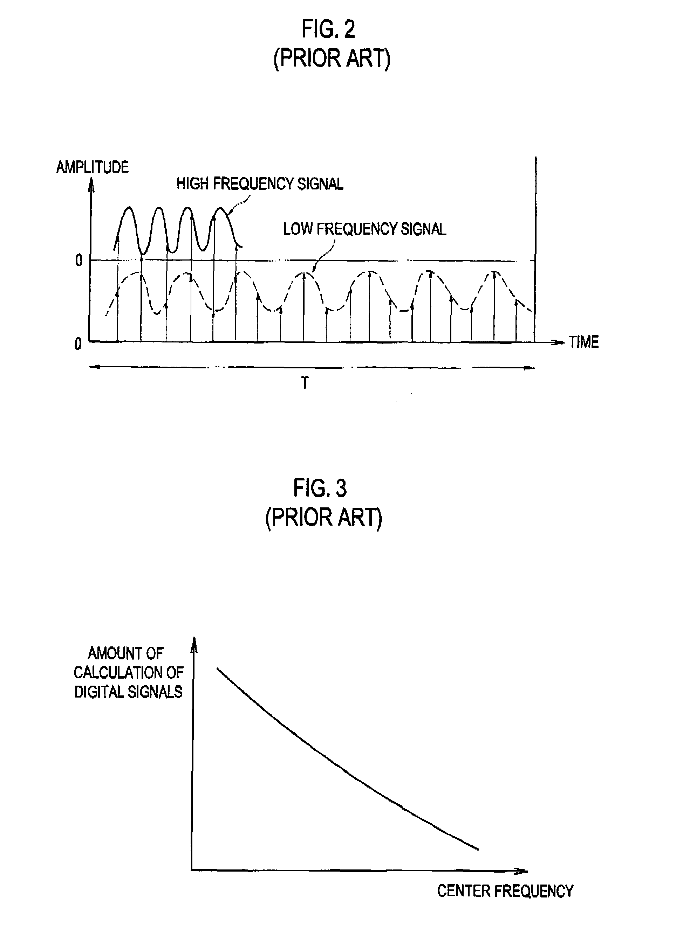

Generally, the electrical signals and the ultrasound signals, which are reciprocally converted in the probe, consist of wide bandwidth signals. The ultrasound signals propagated into a medium are attenuated due to absorption, scattering and reflection. As a center frequency of the ultrasound signals is higher, the attenuation of the ultrasound signals increases. An analog-to-digital converter (ADC) of the conventional ultrasound diagnostic system samples input signals at a constant interval and then converts the sampled signals into digital signals, regardless of a center frequency of the input signals as illustrated in Fig. 2. Therefore, in case of sampling the input signals at a sampling rate determined according to Nyquist theorem to prevent generation of aliasing, if the center frequency is relatively low, then the amount of the digital signals becomes relatively large. On the contrary, if the center frequency is relatively high, then the amount of the digital signals becomes relatively small (see Fig. 3). Thus, data processing capability of a processor for processing the digital signals, which are outputted from the ADC, has to be controlled according to the center frequency of the analog signals. As such, it becomes difficult to design the ultrasound diagnostic system.

**BRIEF DESCRIPTION OF THE DRAWINGS**

Arrangements and embodiments may be described in detail with reference to the following drawings in which like reference numerals refer to like elements and wherein:

Fig. 1 is a schematic diagram illustrating an example of explaining a transmission/reception focusing method of ultrasound signals in a probe using an array transducer;

FIG. 2 is a schematic diagram illustrating an example of sampling a high frequency signal and a low frequency signal according to the prior art;

FIG. 3 is a graph showing a relation between an amount of calculation of digital signals and a center frequency according to the prior art;

FIG. 4 is a block diagram showing a ultrasound diagnostic system in accordance with one embodiment of the present invention;

FIG. 5 is a block diagram showing a beam former in accordance with one embodiment of the present invention;

FIG. 6 is a schematic diagram showing an example of delaying digital signals by using a dual port random access memory in accordance with one embodiment of the present invention;

FIG. 7 is a block diagram illustrating an extraction unit and an interpolation unit for adjusting an amount of digital signals in accordance with one embodiment of the present invention;

FIG. 8 is a diagram showing an example of extracting a part of digital signals in accordance with one embodiment of the present invention;

FIG. 9 is a graph showing a relation between an amount of calculation of digital signals and a center frequency in accordance with one embodiment of the present invention; and

FIG. 10 is a block diagram showing an ultrasound diagnostic system in accordance with another embodiment of the present invention.

## DETAILED DESCRIPTION

The present invention provides an ultrasound diagnostic system and a method for uniformly controlling an amount of calculation of digital signals outputted from a beam former. Hereinafter, one embodiment of the present invention will be described with reference to the accompanying drawings.

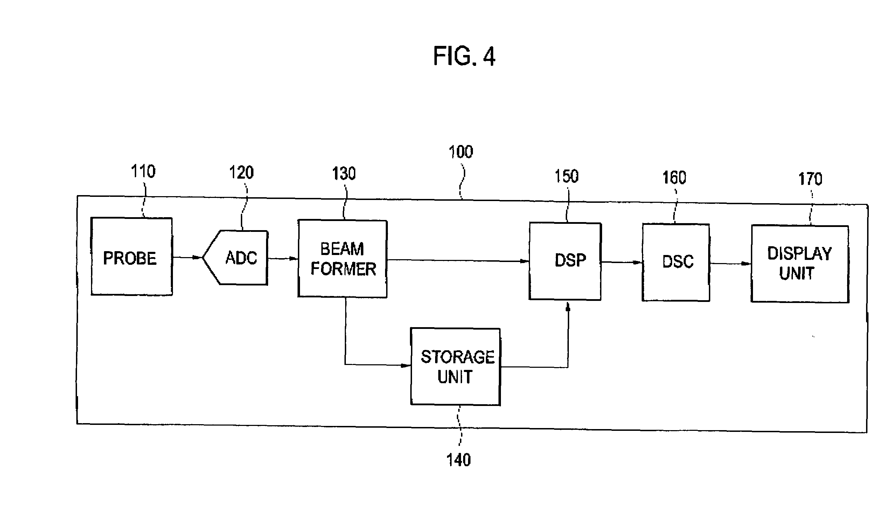

As shown in Fig. 4, the ultrasound diagnostic system 100 includes a probe 110, an analog-to-digital converter (ADC, 120), a beam former 130, a storage unit 140, a digital signal processor (DSP, 150), a digital scan converter (DSC, 160) and a display unit 170.

The probe 110 includes at least one transducer element for converting transmission signals into ultrasound signals to be transmitted to a target object and receiving ultrasound signals reflected from the target object. The transducer element converts the received ultrasound signals into electrical signals of an analog form (hereinafter referred to as analog receive signals). The analog receive signals outputted from the probe 110 are broad bandwidth signals, wherein a center frequency of the analog receive signals reflects characteristics of the transducer element and tissues of the target object.

The ADC 120 samples the analog receive signals outputted from the probe 110 at a constant sampling rate (e.g., 60 MHz) and then converts the sampled signals into digital signals. Since the sampling is carried out at the constant sampling rate by the ADC 120 regardless of the center frequency of the analog receive signals, if the center frequency of the analog receive signals is relatively low, then a relatively large amount of digital signals is obtained. On the contrary, if the center frequency of the analog receive signals is relatively high, then a relatively small amount of digital signals is obtained. When the probe 110 has a plurality of transducer elements, the ADC 120 is installed as many as the number of the transducer elements. That is, the ADC 120 is matched with the transducer element one by one.

The beam former 130 extracts a part of the digital signals at a rate of integer multiples of the center frequency and then forms a receive beam based on the extracted digital signals. That is, the beam former 130 converts the digital signals outputted from the ADC 120 into the constant number of digital signals. The storage unit 140 stores the digital signals outputted from the beam former 130. The DSP 150 processes the digital signals outputted from the beam former 130 or stored in the storage unit 140, thereby forming image data for expressing B, C or D mode images. DSC 160 scan converts the image data inputted from the DSP 150 for display The display unit 160 receives the scan-converted image data and then displays an ultrasound image based on the scan-converted image data.

The beam former 130 delays the digital signals sampled at the constant sampling rate by the ADC 120 and then extracts the sampled digital signals at a rate corresponding to integer multiples of the center frequency of the analog receive signals. This adjusts the amount of the digital signals. Further, the beam former 130 interpolates the extracted digital signals.

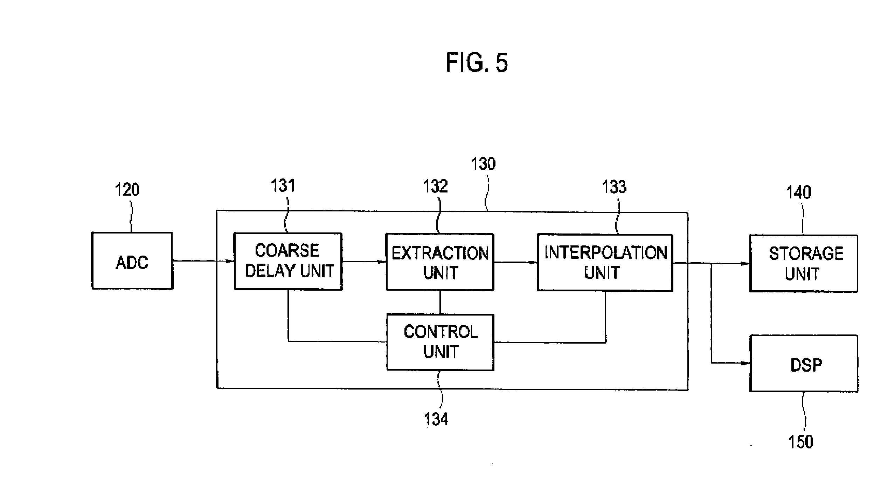

As shown in Fig. 5, the beam former 130 includes a coarse delay unit 131, an extraction unit 132, an interpolation unit 133 and a control unit 134. The extraction unit 132 controls such that the digital signals outputted from the ADC 120 arc maintained in a uniform amount. The beam former 130 controls the operations of the coarse delay unit 131, the extraction unit 132 and the interpolation unit 133. Further, the beam former 130 includes a transmission beam forming unit (not shown) and a receive beam forming unit (not shown) for performing the basic functions of the beam former 130. Also, the beam former 130 may include a gain adjusting unit for compensating the attenuation of the ultrasound signals reflected from the target object.

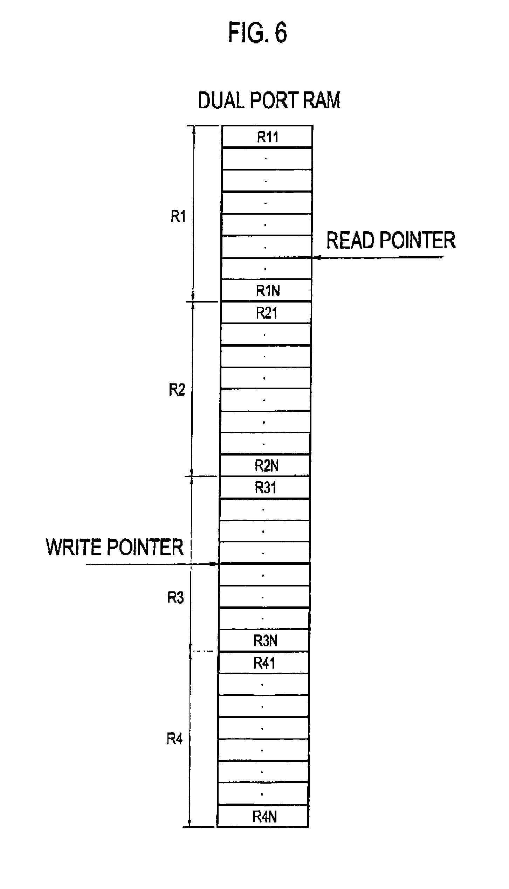

The coarse delay unit 131 may be preferably configured with a dual port random access memory (RAM). As illustrated in Fig. 6, the dual port RAM includes a plurality of memory regions. Each memory region of the dual port RAM is pointed by a write pointer and a read pointer. The dual port RAM includes a writing pin and a reading pin (not denoted)" Data inputted through the writing pin are stored in the memory region pointed by the write pointer. The data stored in the memory region pointed by the read pointer are read out through the reading pin The digital signals outputted from the ADC 120 corresponding to each transducer element are stored in the memory regions R1-R4, which are classified according to each transducer element. Each memory region is partitioned into a plurality of sub memory regions R11-R4N. The digital signals corresponding to an identical transducer element are stored as the type of a digital signal stream. Before the digital signal stream of each transducer element is written in the dual port RAM, two pointers are initialized to point the same position, e.g., a sub memory region R11. The digital signal stream of each transducer element is stored in the memory regions pointed by the write pointer. Each memory region, which stores the digital signal stream to be read out, is pointed by the read pointer after a predetermined time elapses from a time which the digital signal stream is stored in the corresponding memory region or from a time which the corresponding memory region is pointed by the write pointer. The predetermined time is determined according to a delay profile formed by reflecting a distances between each transducer element and the focal point. As mentioned above, the digital signal stream, which is sampled at the constant sampling rate, is coarsely delayed in the coarse delay unit 131.

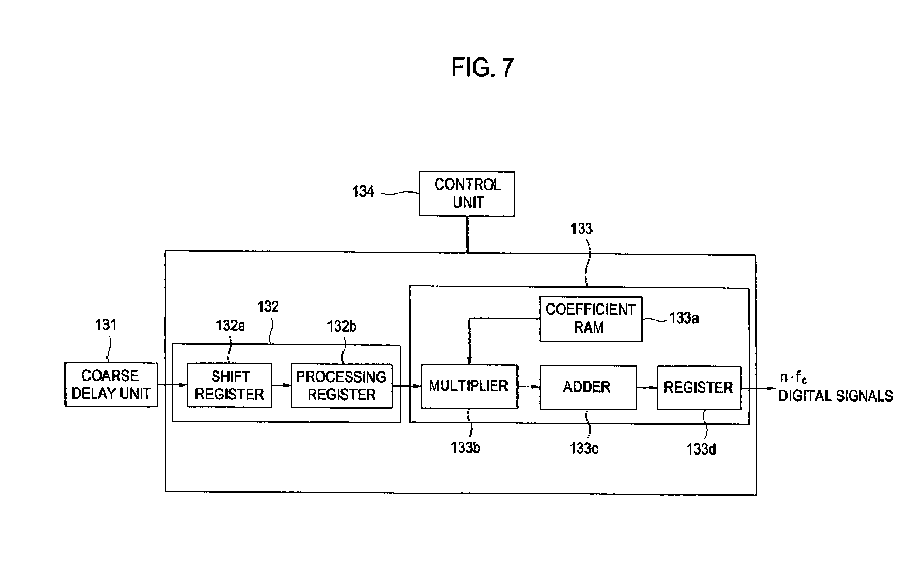

As shown in Fig. 7, the extraction unit 132 includes a shift register 132a and a processing register 132b. The shift register 132a and the processing register 132b are installed as many as the number of the transducer elements The extraction unit 132 adjusts the amount of the digital signals with reference to a preset center frequency of the analog receive signals. In accordance with another embodiment of the present invention, the ultrasound diagnostic system 100 further includes a center frequency information providing unit for analyzing the analog receive signals outputted from the probe 110 so as to provide center frequency information to the extraction unit 132 or the control unit 134.

The digital signal stream of the memory region, which is pointed by the read pointer under the control of the control unit 134, is transferred to the shift register 132a. The digital signal stream corresponding to each transducer element is preferably transferred to the shift register corresponding to the transducer element. Thereafter, a part of the digital signal stream, which is stored in each shifter register 132a at a rate of n times of the center frequency of the analog receive signal received from the probe 110, is extracted under the control of the control unit 134, wherein n is a positive integer. The extracted digital signals are transferred to the processing register 132b. The digital signal stream is preferably extracted at a rate (DR) defined by the following equation (1).

$$DR = fc \times n \qquad\qquad (1)$$

Wherein fc represents the center frequency of the analog receive signal outputted from the probe 110 and n denotes a positive integer in equation (1) It is preferable that n is 4 to extract the digital signals at a rate of at least 2 times of a maximum frequency according to Nyquist theorem for reducing aliasing. However, this positive integer n is not limited.

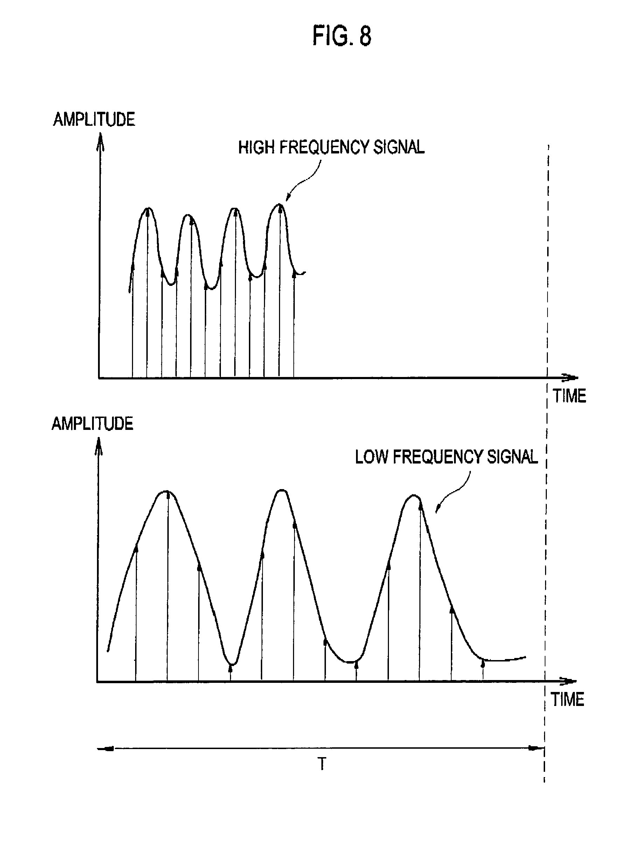

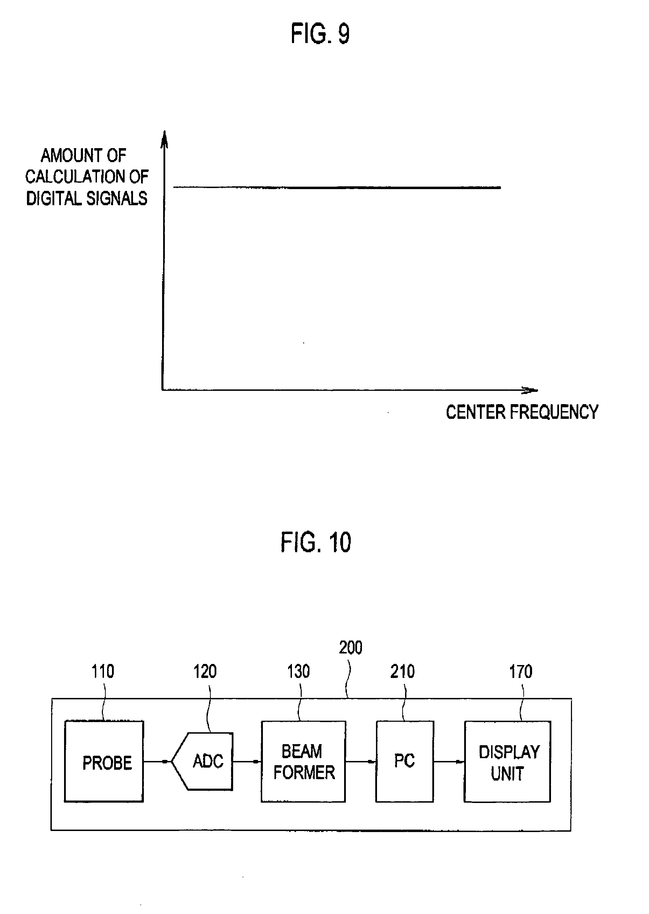

The digital signal stream, which is stored in the shift register 132a, is extracted at a rate of n times of the center frequency of the analog receive signal in accordance with one embodiment of the present invention. Therefore, when a relatively small amount of digital signals is outputted from the ADC 120, the digital signal stream is extracted at a relatively high rate" On the contrary, when a relatively large amount of digital signals is outputted from the ADC 120, the digital signal stream is extracted at a relatively low rate. That is, when assuming that a density of scan lines and a frame rate are identical per each frame, and if the center frequency of the analog receive signal is relatively high (high frequency), then the digital signal stream is extracted at a relatively high rate as illustrated in Fig. 8. On the contrary, when the center frequency of the analog receive signal is relatively low (low frequency), the digital signal stream is extracted at a relatively low rate. Thus, the beam former 130 can output the uniform amount of digital signals regardless of the center frequency of the analog receive signals outputted from the probe 110, as shown in Fig. 9. For the sake of convenience, the waveforms of the high frequency and the low frequency are illustrated in an analog form. However, the digital signals can be extracted from the high frequency and the low frequency signals of a digital form.

The interpolation unit 133 interpolates the digital signals outputted from the processing register 132b. The interpolation unit 133 includes a coefficient RAM 133a, a multiplier 133b, an adder 133c and a register 133d. The coefficient RAM 133a provides a look-up table of interpolation filter coefficients. The multiplier 133b multiplies the digital signals, which are inputted from the processing register 132b, by the interpolation filter coefficients stored in the coefficient RAM 113a The adder 133b adds the output signals of the multiplier 133b. The output signals of the adder 133c, i.e., a receive beam, is stored in the register 133d.

As shown in Fig. 10, the ultrasound diagnostic system 200 may include a personal computer (PC) 210 instead of the DSP 150 and the DSC 160 shown in Fig. 4 in accordance with another embodiment of the present invention. That is, the functions of the DSP and the DSC may be implemented by software in the PC 210. In accordance with the present invention, since the beam former 130 outputs a uniform amount of digital signals regardless of the center frequency of the analog receive signals outputted from the probe 110, the functions of DSP and DSC can be implemented in the PC 210 having a predetermined signal processing capability.

As mentioned above, since the beam former outputs an uniform amount of the digital signals by extracting the digital signals at the rate of integer multiples of the center frequency, there is a merit in that the processing capability of the image processing unit such as DSP or PC does not have any influence on designing the ultrasound diagnostic system in accordance with the present invention.

In accordance with one aspect of the present invention, an ultrasound diagnostic system, includes: a probe for providing electrical analog signals of a predetermined bandwidth in response to ultrasound signals reflected from a target object; an analog-to-digital converter for converting the electrical analog signals to digital signals; and a beam former for extracting a part of digital signals at a rate of n times of a center frequency of the electrical analog signals to form a receive beam, wherein n is a positive integer.

In accordance with another embodiment of the present invention, a method of adjusting an output of digital signals in an ultrasound diagnostic system, includes: a) providing electrical analog signals of a predetermined bandwidth in response to ultrasound signals reflected from a target object b) converting the analog signals to digital signals; and c) extracting a part of digital signals at a rate of n times of a center frequency of the analog signals, wherein n is a positive integer.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1. An ultrasound diagnostic system, comprising:

   a probe for providing electrical analog signals of a predetermined bandwidth in response to ultrasound signals reflected from a target object;
   an analog-to-digital converter for converting the electrical analog signals to digital signals; and
   a beam former for extracting a part of digital signals at a rate of n times of a center frequency of the electrical analog signals to form a receive beam, wherein n is a positive integer.

2. The ultrasound diagnostic system of Claim 1, wherein the beam former includes an extracting unit for extracting a part of digital signals at the rate to adjust an amount of the digital signals.

3. The ultrasound diagnostic system of Claim 2, wherein the extracting unit includes:

   a shift register for storing the digital signals inputted from the analog-to-digital converter; and
   a processing register for extracting a part of digital signals at the rate from the shift register

4. The ultrasound diagnostic system of Claim 3, wherein the beam former further includes an interpolation unit for interpolating the extracted digital signals.

5. The ultrasound diagnostic system of Claim 4, wherein the interpolation unit includes:

   a coefficient random access memory (RAM) for storing a look-up table of interpolation filter coefficients;
   a multiplier for multiplying the digital signals outputted from the processing register by the interpolation filter coefficient to interpolate the digital signals; and
   an adder for adding the interpolated digital signals.

6. The ultrasound diagnostic system of Claim 2, wherein the probe includes a plurality of transducer elements and the beam former includes a delay unit for delaying the electrical analog signals corresponding to respective transducer elements to reflecting positions of the transducer elements.

7. The ultrasound diagnostic system of Claim 6, wherein the delay unit includes a dual port RAM.

8. A method of adjusting an output of digital signals in an ultrasound diagnostic system, comprising.

   a) providing electrical analog signals of a predetermined bandwidth in response to ultrasound signals reflected from a target object;
   b) converting the analog signals to digital signals; and
   c) extracting a part of digital signals at a rate of n times of a center frequency of the electrical analog signals, wherein n is a positive integer

9. The method of Claim 8, wherein the step c) includes:

   c1) storing the digital signals; and
   c2) extracting a part of digital signals at the rate.

10. The method of Claim 9, wherein the step c) further includes c3) interpolating the extracted digital signals.

11. The method of Claim 10, wherein the step c3) includes:

   c31) providing a look-up table of interpolation coefficients;
   c32) multiplying the extracted digital signals by the interpolation filter coefficient to interpolate the digital signals; and
   c33) adding the interpolated digital signals.

# FIG. 1
# (PRIOR ART)

# FIG. 2
# (PRIOR ART)

# FIG. 3
# (PRIOR ART)

# FIG. 4

EP 1 860 460 A1

## FIG. 5

# FIG. 6

DUAL PORT RAM

## FIG. 7

# FIG. 8

AMPLITUDE

HIGH FREQUENCY SIGNAL

TIME

AMPLITUDE

LOW FREQUENCY SIGNAL

TIME

T

# FIG. 9

# FIG. 10

## EUROPEAN SEARCH REPORT

**European Patent Office**

Application Number

EP 07 01 0111

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 042 547 A (WRIGHT J NELSON [US] ET AL) 28 March 2000 (2000-03-28) | 1,2,8 | INV. G01S15/89 |
| Y | * column 2, lines 3-16 * <br> * column 9, lines 33,34 * <br> * column 9, line 63 - column 10, line 16 * <br> * column 16, line 16 - column 18, line 50 * <br> * figures 2b,3 * <br> ----- | 3-7,9-11 | G01S7/52 <br> A61B8/00 <br> G10K11/34 |
| Y | WO 2006/042067 A (UNIV VIRGINIA [US]; HOSSACK JOHN A [US]; BLALOCK TRAVIS N [US]; WALKER) 20 April 2006 (2006-04-20) <br> * page 11, paragraph 4 - page 12, paragraph 1 * <br> ----- | 3-5,9-11 | |
| Y | US 5 469 851 A (LIPSCHUTZ DAVID [US]) 28 November 1995 (1995-11-28) <br> * column 12, lines 5-37 * <br> * figure 4 * <br> ----- | 6,7 | |

**TECHNICAL FIELDS SEARCHED (IPC)**

G01S
A61B
G10K

The present search report has been drawn up for all claims

| Place of search | Date of completion of the search | Examiner |
|---|---|---|
| Munich | 20 August 2007 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 01 0111

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2007

| Patent document cited in search report | | Publication date | Patent family member(s) | | Publication date |
|---|---|---|---|---|---|
| US 6042547 | A | 28-03-2000 | NONE | | |
| WO 2006042067 | A | 20-04-2006 | US | 2006100516 A1 | 11-05-2006 |
| US 5469851 | A | 28-11-1995 | EP | 0696792 A2 | 14-02-1996 |
| | | | JP | 3759769 B2 | 29-03-2006 |
| | | | JP | 8056944 A | 05-03-1996 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020060046253
- KR 1020060069989